# EUROPÄISCHE PATENTSCHRIFT

(11) **EP 2 903 961 B1**
(45) Veröffentlichungstag und Bekanntmachung des Hinweises auf die Patenterteilung: **09.11.2016**
(21) Anmeldenummer: 13766949.5
(22) Anmeldetag: 23.09.2013
(51) Int. Cl.: C07C 67/08, C07C 67/54

(54) **KONTINUIERLICHES VERFAHREN ZUR HERSTELLUNG VON (METH)ACRYLATEN VON C10-ALKOHOLGEMISCHEN**
CONTINUOUS PROCESS FOR THE PREPARATION OF (METH)ACRYLATES OF MIXTURES OF C10-ALCOHOLS
PROCÉDÉ CONTINU POUR LA PRÉPARATION DE (MÉTH)ACRYLATES DE MÉLANGES D'ALCOOLS EN C10

(30) Priorität: 01.10.2012 DE 102012217943
(43) Veröffentlichungstag der Anmeldung: 12.08.2015
(73) Patentinhaber: BASF SE, 67056 Ludwigshafen am Rhein (DE)
(72) Erfinder: JÄGER, Ulrich, 67354 Römerberg (DE); SCHLIEPHAKE, Volker, 67105 Schifferstadt (DE); LANG, Ortmund, 66909 Quirnbach (DE); PETZOLDT, Jochen, 79595 Ruemmingen (DE); KORN, Tobias Johannes, 67061 Ludwigshafen (DE); BETTE, Virginie, 68199 Mannheim (DE); HECHLER, Claus, 67063 Ludwigshafen (DE)
(74) Vertreter: BASF IP Association
(86) Internationale Anmeldenummer: PCT/EP2013/069717
(87) Internationale Veröffentlichungsnummer: WO 2014/053347

(56) Entgegenhaltungen:
- WO-A1-98/56746
- WO-A1-2009/106550
- DE-A1-102005 006 974

## Beschreibung

Die Erfindung betrifft ein kontinuierliches Verfahren zur Herstellung von (Meth)acrylaten von C₁₀-Alkoholgemischen durch Veresterung von (Meth)acrylsäure mit einem Isomerengemisch von C₁₀-Alkoholen aus 2-Propylheptanol als Hauptisomer und mindestens einem der C₁₀-Alkohole 2-Propyl-4-methylhexanol, 2-Propyl-5-methylhexanol, 2-Isopropylheptanol, 2-Isopropyl-4-methylhexanol, 2-isopropyl-5-methylhexanol und/oder 2-Propyl-4,4-dimethylpentanol. Der Begriff (Meth)acrylsäure steht in dieser Schrift verkürzend für Methacrylsäure und/oder Acrylsäure, (Meth)acrylsäureester für Methacrylsäureester und/oder Acrylsäureester bzw. (Meth)acrylat für Methacrylat und/oder Acrylat.

Das Isomerengemisch von C₁₀-Alkoholen besteht aus 2-Propylheptanol als Hauptisomer und mindestens einem der C₁₀-Alkohole 2-Propyl-4-methylhexanol, 2-Propyl-5-methylhexanol, 2-Isopropylheptanol, 2-Isopropyl-4-methylhexanol, 2-isopropyl-5-methylhexanol und/oder 2-Propyl-4,4-dimethylpentanol, wobei diese Verbindungen, inklusive 2-Propylheptanol, nachstehend zur Abkürzung als "Propylheptanol-Isomere" bezeichnet werden. Die daraus hergestellten (Meth)acrylate werden in dieser Schrift abkürzend als "Propylheptyl(meth)acrylate" bezeichnet. Die auf Basis von (Meth)acrylaten von Propylheptanol-Isomeren hergestellten Polymere beziehungsweise Copolymere sind in Form von Polymerdispersionen von großer wirtschaftlicher Bedeutung. Sie finden beispielweise Anwendung als Klebstoffe, Anstrichmittel oder Textil-, Leder- und Papierhilfsmittel.

Aus der DE 100 36 879 A1 ist bereits ein kontinuierliches Verfahren zur Herstellung von Estern der (Meth)acrylsäure durch Veresterung der (Meth)acrylsäure mit C₆-C₁₀-Alkanolen bekannt. Die Herstellung von (Meth)acrylaten, insbesondere 2-Ethylhexylacrylat, ist ebenfalls in DE 102 46 869 A1 beschrieben.

Aus der DE 10 2005 006974 A1 ist ein kontinuierliches Verfahren zur Herstellung von Cyclohexyl(meth)acrylat bekannt, welches folgende Verfahrensschritte umfasst: Veresterung, Neutralisation, Wäsche des Roh-(meth)acrylats, Schleppmitteldestillation des Roh-(Meth)acrylats und Leichtsiederabtrennung aus dem Sumpfstrom dieser Schleppmitteldestillation sowie eine Reindestillation und Rückstandsdestillation.

Die WO 98/56746 A1 offenbart die Verwendung von Diestern der Sebacinsäure mit 4-Hydroxy-2,2,6,6-tetramethylpiperidin-N-oxyl ("Prostab^{®} 5415") in Nitroxydmischungen mit synergistischer Wirkung zur Inhibierung der Polymerisation von Acrylaten.

Aus der WO 01/60779 A1 ist die kontinuierliche Herstellung von (Meth)acrylaten von C₆-C₁₂-Alkanolen, Cyclopentanol oder Cyclohexanol bekannt.

Darüber hinaus wird die diskontinuierliche Herstellung von (Meth)acrylaten von C₁₀-Alkoholgemischen in EP 2 257 519 A1 beschrieben.

Die im Stand der Technik beschriebenen kontinuierlichen Verfahren haben insbesondere den Nachteil, dass durch die mehrfache Destillation des Zielesters der Energieaufwand besonders hoch ist und dass durch die thermische Belastung Produktverluste sowie die Bildung von Nebenkomponenten durch Zersetzung des Zielesters auftreten können. Zudem sorgt die mehrfache Erhitzung zu unerwünschten Nebenreaktionen wie beispielsweise Polymerisation.

Es war demgegenüber Aufgabe der Erfindung, ein Verfahren zur Herstellung von Propylheptyl(meth)acrylaten durch direkte Veresterung von (Meth)acrylsäure mit C₁₀-Alkoholgemischen mit 2-Propylheptanol als Hauptisomer zur Verfügung zu stellen, das die Nachteile des Standes der Technik nicht aufweist, und insbesondere einen qualitativ hochwertigen Zielester in einem energetisch günstigen Verfahren zur Verfügung stellt.

Die Aufgabe wurde gelöst durch ein kontinuierliches Verfahren zur Herstellung von (Meth)acrylaten von C₁₀-Alkoholgemischen durch Umsetzung von Rein-(Meth)acrylsäure mit einem Isomerengemisch von C₁₀-Alkoholen aus 2-Propylheptanol als Hauptisomer und mindestens einem der C₁₀-Alkohole 2-Propyl-4-methylhexanol, 2-Propyl-5-methylhexanol, 2-Isopropylheptanol, 2-Isopropyl-4-methylhexanol, 2-isopropyl-5-methylhexanol und/oder 2-Propyl-4,4-dimethylpentanol, in Gegenwart mindestens eines sauren Katalysators und mindestens eines Polymerisationsinhibitors und in Gegenwart eines Schleppmittels für das Veresterungswasser, mit folgenden Verfahrensschritten:
- Veresterung der Rein-(Meth)acrylsäure (1) mit dem Isomerengemisch aus Propylheptanol-Isomeren (2) in Gegenwart des sauren Katalysators (3), des Polymerisationsinhibitors (4) sowie des Schleppmittels für das Veresterungswasser (5) in einer Reaktionszone (A), wobei das Veresterungswasser in einer der Reaktionszone (A) aufgesetzten Destillationszone als Azeotrop mit dem Schleppmittel abgezogen wird und wobei ein Reaktionsaustrag erhalten wird (Verfahrensstufe A), der
- einer Neutralisation zugeführt wird, wobei aus dem Reaktionsaustrag aus der Veresterung mittels einer alkalischen Lösung der saure Katalysator (3) sowie nicht umgesetzte Rein-(Meth)acrylsäure (1) neutralisiert werden unter Erhalt von Roh-Propylheptyl(meth)acrylat (Verfahrensstufe B),
- Wäsche des Roh-Propylheptyl(meth)acrylats aus Verfahrensstufe B, wobei Reste von Salzen aus dem Roh-Propylheptyl(meth)acrylat abgetrennt werden (Verfahrensstufe C),
- Schleppmitteldestillation des gewaschenen Roh-Propylheptyl(meth)acrylats aus Verfahrensstufe C unter vermindertem Druck und unter kontinuierlicher Zudosierung mindestens eines Polymerisationsinhibitors, wobei das Schleppmittel, andere Leichtsieder sowie ein sehr kleiner Anteil an Propylheptyl(meth)acrylat abgezogen werden (Verfahrensstufe D),
- Leichtsiederabtrennung aus dem Sumpfstrom aus der Verfahrensstufe D unter vermindertem Druck und kontinuierlicher Zudosierung mindestens eines Polymerisationsinhibitors zur Abtrennung der Reste an Leichtsiedern sowie eines kleinen Anteils des Propylheptyl(meth)acrylats (Verfahrensstufe E),
- Luftsättigung des Sumpfstroms aus Verfahrensstufe E im Gegenstrom durch Einblasen eines sauerstoffhaltigen Gasgemisches und Abtrennung des entstehenden Abgases(Verfahrensstufe F),
- Reindestillation des Sumpfstroms aus der Verfahrensstufe F unter vermindertem Druck und kontinuierlicher Zudosierung mindestens eines Polymerisationsinhibitors unter Erhalt von Rein-Propylheptyl(meth)acrylat sowie eines Sumpfstromes, enthaltend die Polymerisationsinhibitoren sowie Hochsieder (Verfahrensstufe G);
- Rückstandsdestillation des Sumpfstroms aus der Verfahrensstufe G unter vermindertem Druck und kontinuierlicher Zudosierung mindestens eines Polymerisationinhibitors, wobei restliche Anteile an Propylheptyl(meth)acrylat von Polymerisationsinhibitoren sowie Hochsiedern getrennt werden (Verfahrensstufe H).

Das erfindungsgemäße Verfahren ist im Vergleich zu den bekannten Verfahren besonders vorteilhaft, da bei der Durchführung der bekannten Verfahrensweise sich während der Reindestillation des Rohesters Polymer bildete. Die Bildung des Polymers kann nun durch Beladung mit einem sauerstoffhaltigem Gas (Sauerstoff, Luft oder Magerluft) vermieden werden. Diese Luftsättigung wird als Verfahrensstufe F weiter unten näher beschrieben.

Das erfindungsgemäße Verfahren geht aus von den Edukten Propylheptanol-Isomeren und Rein-(Meth)acrylsäure.

Als Rein-(Meth)acrylsäure wird vorliegend eine (Meth)acrylsäure-Qualität bezeichnet, die mindestens 98 Gew.-% (Meth)acrylsäure oder auch mit mindestens 99,5 Gew.-% (Meth)acrylsäure, daneben maximal 0,2 Gew.-% Wasser sowie jeweils maximal 0,03 Gew.-% Essigsäure, Propionsäure und Isobuttersäure enthält.

Nach dem erfindungsgemäßen Verfahren wird ein C₁₀-Alkoholgemisch eingesetzt, das als Hauptisomer 2-Propylheptanol enthält. Unter dem Begriff Hauptisomer im Sinne der vorliegenden Erfindung wird ein Gehalt an 2-Propylheptanol von bis zu 100 Gew.-%, bezogen auf das Gesamtgewicht des C₁₀-Alkoholgemisches, verstanden. Der Gehalt an 2-Propylheptanol beträgt im Allgemeinen mindestens 50 Gew.-%, vorzugsweise 60 bis 98 Gew.-% und besonders bevorzugt 80 bis 95 Gew.-%, insbesondere 85 bis 95 Gew.-%, jeweils bezogen auf das Gesamtgewicht des C₁₀-Alkoholgemisches.

Neben 2-Propylheptanol als Hauptisomer enthält das C₁₀-Alkoholgemisch noch mindestens einen der C₁₀-Alkohole 2-Propyl-4-methylhexanol, 2-Propyl-5-methylhexanol, 2-Isopropylheptanol, 2-Isopropyl-4-methylhexanol, 2-Isopropyl-5-methylhexanol und/oder 2-Propyl-4,4-dimethylpentanol. Diese Verbindungen, inklusive 2-Propylheptanol, werden nachstehend zur Abkürzung "Propylheptanol-Isomere" bezeichnet. Die Anwesenheit anderer Isomere der 2-Propylheptanol-Komponente - beispielsweise von denen zu 2-Propylheptanol isomeren Alkoholen 2-Ethyl-2,4-dimethylhexanol, 2-Ethyl-2-methylheptanol und/oder 2-Ethyl-2,5-dimethylhexanol herrührend, in dem C₁₀-Alkoholgemisch ist möglich, aber wenn überhaupt, sind diese nur in Spuren enthalten.

Zur Herstellung von 2-Propylheptanol bzw. Propylheptanol-Isomeren wird an dieser Stelle auf die deutsche Offenlegungsschrift DE 10 2007 001 540 A1 und die darin zitierte Literatur verwiesen.

Geeignete Mischungen von 2-Propylheptanol mit den dazu isomeren Alkoholen umfassen beispielsweise solche aus 60 bis 99 Gew.-% 2-Propylheptanol, 0,1 bis 15 Gew.-% 2-Propyl-4-methylhexanol, 0,1 bis 15 Gew-% 2-Propyl-5-methylhexanol und 0,01 bis 5 Gew.-% 2-Isopropylheptanol, wobei die Summe der einzelnen Bestandteile 100 Gew.-% nicht überschreitet. Bevorzugt addieren sich die Anteile der einzelnen Bestandteile zu 100 Gew.-%.

Bevorzugte C₁₀-Alkoholgemische sind beispielsweise solche aus 80 bis 98 Gew.-% 2-Propylheptanol, 0,5 bis 10 Gew.-% 2-Propyl-4-methylhexanol, 0,5 bis 10 Gew.-% 2-Propyl-5-methylhexanol und 0,1 bis 2 Gew.-% 2-Isopropylheptanol, wobei die Summe der einzelnen Bestandteile 100 Gew.-% nicht überschreitet. Bevorzugt addieren sich die Anteile der einzelnen Bestandteile zu 100 Gew.-%.

Besonders bevorzugte C₁₀-Alkoholgemische sind solche aus 90 bis 95 Gew.-% 2-Propylheptanol, 2 bis 5 Gew.-% 2-Propyl-4-methylhexanol, 2 bis 5 Gew.-% 2-Propyl-5-methylhexanol und 0,1 bis 1 Gew.-% 2-Isopropylheptanol, wobei die Summe der einzelnen Bestandteile 100 Gew.-% nicht überschreitet. Bevorzugt addieren sich die Anteile der einzelnen Bestandteile zu 100 Gew.-%.

Andere ebenfalls geeignete C₁₀-Alkoholgemische sind in der europäischen Patentanmeldung EP 2 257 519 A1, ab Seite 3, Zeile 30 bis Seite 4, Zeile 17, beschrieben.

Die Mischungen aus 2-Propylheptanol und den dazu isomeren Alkoholen können als Verunreinigungen bedingt durch das Herstellverfahren in Spuren noch n-Pentanol, 2-Methylbutanol und/oder 3-methylbutanol enthalten. Die Gehalte an diesen Alkoholen liegen im Allgemeinen bei jeweils maximal 0,5 Gew.-%, bezogen auf das Gesamtgewicht des C₁₀-Alkoholgemisches.

Die Zusammensetzung der (Meth)acrylate von C₁₀-Alkoholgemischen, die nach dem erfindungsgemäßen Verfahren hergestellt werden, entspricht praktisch der Zusammensetzung der zu ihrer Herstellung bei der Veresterung verwendeten Propylheptanol-Isomerengemische.

Das erfindungsgemäße Verfahren ist vorteilhaft, da ein hoher Veresterungsgrad erreicht wird und hohe Ausbeuten in einem energetisch günstigen Verfahren erzielt werden. Weiterhin tritt keine wesentliche Polymerbildung auf.

Die einzelnen Verfahrensstufen werden im Folgenden beschrieben.

### Veresterung der Rein-(Meth)acrylsäure mit dem Isomerengemisch aus Propylheptanol-Isomeren (Verfahrensstufe A)

Die Edukte Rein-(Meth)acrylsäure (1) und Isomerengemisch aus Propylheptanol-Isomeren (2) werden kontinuierlich einer geeigneten Reaktionszone zugeführt, die sowohl ein einzelner Reaktor als auch eine Kaskade von zwei oder mehreren hintereinander geschalteten Reaktionsbereichen sein kann, wobei dann der Austragsstrom eines Reaktionsbereiches den Zulaufstrom des nächstfolgenden Reaktionsbereiches bildet. In der Ausführungsform mit mehreren Reaktionsbereichen werden bevorzugt die aufsteigenden Dämpfe aus sämtlichen Reaktionsbereichen einer einzigen Destillationseinrichtung zugeführt, deren flüssiger Ablauf nur in den ersten Reaktionsbereich zurückgeführt wird.

Die Reaktionseinheiten können getrennte Reaktoren sein oder auch unterschiedliche Bereiche in einem Reaktor.

Als Reaktoren können mit Heizspiralen oder Doppelmantel ausgerüstete Rührkessel oder Blasen mit außenliegendem Naturumlauf- oder Zwangsumlaufverdampfer eingesetzt werden.

Auf den ersten Reaktor ist eine Destillationskolonne für die Abtrennung des Veresterungswassers aufgesetzt.

Vorteilhaft können die aufsteigenden Brüden aus allen Reaktionsbereichen einer einzigen Destillationskolonne zugeführt werden, deren Ablauf nur in den ersten Reaktionsbereich zugeführt wird. Es ist jedoch auch möglich, alle Reaktionsbehälter mit jeweils einer eigenen aufgesetzten Destillationskolonne auszustatten.

Die Destillationskolonne kann eine Füllkörperkolonne, eine Packungskolonne oder eine Bodenkolonne, bevorzugt mit 1 bis 15 theoretischen Trennstufen, sein.

Rein-(Meth)acrylsäure und das Isomerengemisch aus Propylheptanol-Isomeren werden bevorzugt in einem Molverhältnis im Bereich von 0,9 bis 2,0, insbesondere im Bereich von 1,05 bis 1,15, eingesetzt.

Als saure Veresterungskatalysatoren (3) eignen sich insbesondere Schwefelsäure, Paratoluolsulfonsäure oder andere organische Sulfonsäuren, besonders Methansulfonsäure, Benzolsulfonsäure oder Dodecylbenzolsulfonsäure.

Der saure Veresterungskatalysator wird vorzugsweise in einer Konzentration von 1 bis 5 Gew.-%, bezogen auf die eingesetzte Rein-(Meth)acrylsäure zugeführt.

Als Schleppmittel für das Veresterungswasser (5) wird bevorzugt eine Substanz oder ein Gemisch von Substanzen, ausgewählt aus der nachfolgenden Aufzählung eingesetzt: Cyclohexan, Cyclohexen, Methylcyclohexan, Benzol, Toluol, Hexane, Hexene, Heptane oder Heptene sowie Derivate davon wie Propylhepten.

Als Polymerisationsinhibitor (4) wird eine Substanz oder eine Mischung von Substanzen in einer Konzentration im Bereich von 100 bis 5000 ppm, bezogen auf den Austrag aus der Reaktionszone, ausgewählt aus der nachfolgenden Aufzählung: Phenothiazin, 4-Nitrosophenol, 4-Hydroxy-2,3,6,6-Tetramethylpiperidin-N-Oxyl, Hydrochinon oder Hydrochinonmonomethylether, eingesetzt. In einer bevorzugten Variante des erfindungsgemäßen Verfahrens werden N-Oxyl-Gruppen-haltige Stabilisatoren mit einem niedrigeren Dampfdruck als die der zuvor genannten Stabilisatoren eingesetzt. Diese Stabilisatoren werden weiter unten näher beschrieben.

Vorteilhaft kann zusätzlich als Polymerisationsinhibitor Sauerstoff eingesetzt werden.

Die Umsetzung in Verfahrensstufe A erfolgt entweder im Vakuum oder unter Normaldruck bei einer Temperatur zwischen 70 und 140 °C (Normaldruck), bevorzugt zwischen 100 und 130 °C (Normaldruck). Vorzugsweise sind Druck und Temperaturbereich in allen Reaktionsbereichen der Veresterung (Verfahrensstufe A) gleich.

Der Reaktionszone sind eine oder mehrere Destillationskolonnen aufgesetzt. Das darin anfallende Destillat wird kondensiert und in einem Phasentrenner in eine organische und eine wässrige Phase getrennt. Die wässrige Phase wird entweder ins behandlungsbedürftige Abwasser gegeben oder vorzugsweise der Wäsche in Verfahrensstufe C zugeführt.

Die organische Phase, die das Schleppmittel enthält, wird als Rücklauf auf die Destillationskolonne(n) und gegebenenfalls auch direkt in die Reaktionszone zurückgefahren.

Der Veresterungsaustrag aus der Reaktionszone enthält den Zielester, nicht umgesetzte Edukte, Katalysator, Polymerisationsinhibitor(en) sowie Nebenprodukte.

Als Nebenprodukte kommen insbesondere Propylheptylacetat, Oxyester von Propylheptyl(meth)acrylat und Propylheptanol-Isomeren, Ester der Diacrylsäure mit Propyl-heptanolisomeren sowie Propylheptylpropionat in Frage.

Der Veresterungsaustrag wird vorzugsweise in einem Wärmetauscher auf eine Temperatur von 20 bis 40 °C gekühlt und anschließend der Neutralisation (Verfahrensstufe B) zugeführt.

In der Regel beträgt die Verweilzeit in der Veresterung (Verfahrensstufe A) zwischen 5 und 30 Stunden, bevorzugt zwischen 5 und 15 Stunden.

### Neutralisation (Verfahrensstufe B)

In Verfahrensstufe B wird der Veresterungsaustrag vom Katalysator sowie von nicht umgesetzter (Meth)acrylsäure mit Hilfe einer alkalischen Lösung, insbesondere Natronlauge, Kalilauge oder Natriumcarbonat, befreit.

Die Neutralisation wird vorzugsweise in Mixer-Settlern durchgeführt. Die wässrige Phase wird dem behandlungsbedürftigen Abwasser zugeführt, während die organische Phase der nächsten Verfahrensstufe, der Wäsche (Verfahrensstufe C) zugeführt wird.

### Wäsche (Verfahrensstufe C)

Hierbei wird die organische Phase aus der Neutralisation mit Hilfe einer Waschlösung, insbesondere Wasser, das vorteilhaft Wasser aus dem Phasentrenner der Reaktionszone, das heißt Prozesswasser sein kann, von Salzen befreit. Die wässrige Phase wird vorzugsweise dem behandlungsbedürftigen Abwasser zugeführt. Apparativ wird die Verfahrensstufe C, wie auch die Verfahrensstufe B in Mixer-Settlern, durchgeführt. Als Settler für die Neutralisation (Verfahrensstufe B) wie auch für die Wäsche (Verfahrensstufe C) kommen beispielsweise Dekanter oder Extraktionskolonnen in Frage.

Der bei der Wäsche erhaltene gewaschene Rohester (Neutralester) wird in einer Reihe von Destillationsstufen aufgearbeitet:

### Schleppmitteldestillation (Verfahrensstufe D)

In der Schleppmitteldestillation wird das in der Reaktionszone zur Abtrennung des Veresterungswassers eingesetzte Schleppmittel über Kopf destilliert und vorzugsweise zum großen Teil in die Veresterung (Verfahrensstufe A) zurückgeführt. Ein kleiner Teil des Destillats wird ausgeschleust, um eine Aufpegelung von Verunreinigungen zu vermeiden.

Als Apparate für die Schleppmitteldestillation kommen beispielsweise Füllkörperkolonnen, Packungskolonnen oder Bodenkolonnen mit vorzugsweise 1 bis 5 theoretischen Trennstufen in Frage. Die Schleppmitteldestillation wird vorzugsweise bei einem Kopfdruck zwischen 60 und 150 mbar, besonders bevorzugt bei einem Kopfdruck zwischen 70 und 100 mbar betrieben.

### Leichtsiederabtrennung (Verfahrensstufe E)

In der Leichtsiederabtrennung werden verbleibende Leichtsieder aus dem Sumpfprodukt der Schleppmitteldestillation (Verfahrensstufe D) über Kopf destilliert und vorzugsweise in die Neutralisation (Verfahrensstufe B) oder in die Veresterung (Verfahrensstufe A) recycliert.

Als Apparate für die Leichtsiederabtrennung kommen beispielsweise Füllkörperkolonnen, Packungskolonnen oder Bodenkolonnen, vorzugsweise mit 1 bis 15 theoretischen Trennstufen, in Frage. Die Leichtsiederabtrennung wird vorzugsweise bei einem Kopfdruck von 5 bis 80 mbar, insbesondere bei einem Kopfdruck zwischen 5 und 50 mbar, durchgeführt.

Es ist möglich, die Schleppmitteldestillation (Verfahrensstufe D) und die Leichtsiederabtrennung (Verfahrensstufe E) in einer gemeinsamen Destillationseinheit durchzuführen.

### Luftsättigung (Verfahrensstufe F)

In der Luftsättigung wird das aus dem Sumpfprodukt der Leichtsiederabtrennung (Verfahrensstufe E) erhaltene Rohprodukt im Gegenstrom durch Einblasen eines sauerstoffhaltigen Gasgemisches gesättigt. Das sauerstoffhaltige Gas ist bevorzugt Sauerstoff, Luft oder sog. Magerluft. Besonders bevorzugt beträgt der Sauerstoffgehalt des sauerstoffhaltigen Gases zwischen 1 und 21 Vol.-%, besonders bevorzugt zwischen 4 und 8 Vol.-%, jeweils bezogen auf das Gesamtvolumen des sauerstoffhaltigen Gases.

Die Luftsättigung erfolgt bei Normaldruck bzw. unter einem Druck, der sich entsprechend der eingeleiteten Luftmenge und dem sich daraus ergebenden Druckverlust ergibt. Die Luftsättigung erfolgt im Gegenstrom, wobei die Luft im Gegenstrom zur das Rohprodukt enthaltenden Flüssigkeit mit einer Geschwindigkeit von 0,1 bis 10 m³/h, bevorzugt von 0,5 bis 5 m³/h und besonders bevorzugt von 1 bis 2 m³/h eingeleitet wird. Diese Geschwindigkeitswerte beziehen sich auf eine Magerluft mit ca. 6 Vol.-% Sauerstoffgehalt. Soll eine Gasmischung mit einem niedrigeren oder höheren Sauerstoffgehalt als Magerluft verwendet werden, muss die Einleitungsgeschwindigkeit entsprechend angepasst werden, um eine ausreichende Sättigung mit Sauerstoff der das Rohprodukt enthaltenden Flüssigkeit zu gewährleisten.

Die das Rohprodukt enthaltende Flüssigkeit weist unter dem gegebenen Druck (Normaldruck) beim Einleiten in die Luftsättigung beispielsweise eine Temperatur von 20 bis 80 °C, bevorzugt von 30 bis 50 °C auf. Diese das Rohprodukt enthaltende Flüssigkeit wird mit einer Geschwindigkeit von beispielsweise 300 bis 600 kg/h eingeleitet.

Als Apparate für die Luftsättigung kommen beispielsweise Füllkörperkolonnen, Packungskolonnen oder Bodenkolonnen, vorzugsweise mit 1 bis 15 theoretischen Trennstufen, in Frage. Dabei wird die das Rohprodukt enthaltende Flüssigkeit auf den Kopf der Kolonne gegeben und die Luft in den unteren Teil der Kolonne eingeleitet. Die Einleitung der Luft kann durch ein Einsteckrohr oder durch diverse, dem Fachmann bekannte, Verteilersysteme erfolgen. Der Flüssigkeitsablauf aus dem Sumpf wird - wie unten beschrieben - der Reindestillation (Verfahrensstufe G) zugeführt.

### Reindestillation (Verfahrensstufe G)

In der Reindestillation wird das Rein-Propylheptyl(meth)acrylat aus dem Sumpfstrom der Luftsättigung (Verfahrensstufe F) dampfförmig gewonnen und mit einem Lagerstabilisator stabilisiert. Als Lagerstabilisator kommt beispielsweise Hydrochinonmonomethylether in Frage. Als Destillationsapparate für die Reindestillation kommen beispielsweise Füllkörperkolonnen, Packungskolonnen oder Bodenkolonnen, insbesondere mit 1 bis 15 theoretischen Trennstufen oder auch ein Dünnschichtverdampfer in Frage. Die Reindestillation wird vorzugsweise bei einem Kopfdruck im Bereich von 1 bis 20 mbar, besonders bevorzugt bei einem Kopfdruck im Bereich von 1 bis 5 mbar, betrieben.

### Rückstandsdestillation (Verfahrensstufe H)

In der Rückstandsdestillation werden aus dem Sumpfprodukt der Reindestillation noch enthaltene Anteile des Zielesters Propylheptyl(meth)acrylat über Kopf destilliert und in die Reindestillation zurückgeführt. Als Apparate kommen Füllkörperkolonnen, Packungskolonnen oder Bodenkolonnen, insbesondere mit 1 bis 15 theoretischen Trennstufen, oder auch Dünnschichtverdampfer in Frage. Die Rückstandsdestillation wird vorzugsweise bei einem Kopfdruck im Bereich von 1 bis 20 mbar, besonders bevorzugt bei einem Kopfdruck im Bereich von 1 bis 5 mbar, durchgeführt.

Aus der Reindestillation (Verfahrensstufe G) wird Rein-Propylheptyl(meth)acrylat gewonnen, wobei vorliegend unter Rein-Propylheptyl(meth)acrylat eine Rein-Propylheptyl(meth)acrylat-Qualität mit mindestens 98 Gew.-% Propylheptyl(meth)acrylat, maximal 1000 ppm Wasser, maximal 100 ppm (Meth)acrylsäure, einer Farbzahl < 10 APHA sowie 200 bis 300 +/- 5 ppm Stabilisator wie beispielsweise Hydrochinonmonomethylether verstanden wird.

Die in den einzelnen Destillationsstufen D bis H eingesetzten Destillationsapparate umfassen jeweils einen Verdampfer sowie eine Kondensationseinheit. Die Verdampfer können Naturumlauf- oder Zwangsumlaufverdampfer, Fallfilmverdampfer oder Dünnschichtverdampfer sein. Als Kondensationseinheit kommen beispielsweise Rohrbündelwärmetauscher, Plattenwärmetauscher oder Direktkondensatoren (Quenchapparate) in Frage.

In sämtlichen Verfahrensstufen werden gegen unerwünschte Polymerisation Polymerisationsinhibitoren zugesetzt. Als Polymerisationsinhibitoren kommen die zuvor beschriebenen Stabilisatoren oder auch Gemische derselben in Frage, wie beispielsweise Phenothiazin, 4-Nitrosophenol, 4-Hydroxy-2,2,6,6-tetramethylpiperidin-N-oxyl, Hydrochinon oder Hydrochinonmonomethylether. Diese werden in der Regel in einer Konzentration von 100 bis 5000 ppm bezogen auf die die zu stabilisierende Verbindung eingesetzt.

Jedoch hat sich beim Einsatz von 4-Hydroxy-2,2,6,6-tetramethylpiperidin-N-oxyl bei der Destillation, insbesondere bei der Reindestillation (Verfahrensstufe G) nachteilige Effekte ergeben. Bei dem üblicherweise dort eingesetzten Unterdruck am Kopf der Destillationseinheit von 1 bis 20 mbar, bevorzugt von 1 bis 5 mbar, werden geringe Mengen dieses Stabilisators über Kopf der Kolonne zusammen mit dem Rein-Propylheptyl(meth)acrylat ausgetragen. Dies kann zu negativen Eigenschaften wie Verfärbungen des Rein-Propylheptyl(meth)acrylats sowie den daraus hergestellten Polymeren führen. Insbesondere in Anwendungsbereichen solcher Polymere in Lacken ist dies unerwünscht. Es wurde daher gefunden, dass dieser Nachteil vermieden werden kann, wenn Substanzen mit gleichem Wirkmechanismus, d.h. Substanzen, die ebenfalls die stabilisierende N-Oxyl-Gruppe tragen, aber höherem Molekulargewicht und damit einem niedrigerem Dampfdruck eingesetzt werden.

Dadurch werden insbesondere qualitativ hochwertigen Zielester erhalten, da diese frei von verfärbenden Substanzen sind.

Derartige Polymerisationsinhibitoren enthalten mindestens eine N-Oxyl-Gruppe und haben einen Dampfdruck von weniger als 10⁻⁵ mbar, bevorzugt weniger als 10⁻⁶ mbar, besonders bevorzugt weniger als 10⁻⁸ mbar und ganz besonders bevorzugt weniger als 5*10⁻⁹ mbar.

Bevorzugte Polymerisationsinhibitoren mit mindestens einer N-Oxyl-Gruppe und einem niedrigem Dampfdruck sind Ester von Dicarbonsäuren. Dabei können nur eine, bevorzugt jedoch beide Säuregruppen der Dicarbonsäure mit der N-Oxyl-gruppenhaltigen Verbindung verestert sein.

Als N-Oxyl-gruppenhaltige Verbindung sind z. B. geeignet 4-Hydroxy-2,2,6,6-tetramethyl-piperidin-N-oxyl, 4-Oxo-2,2,6,6-tetramethyl-piperidin-N-oxyl, 4-Methoxy-2,2,6,6-tetramethyl-piperidin-N-oxyl, 4-Acetoxy-2,2,6,6-tetramethyl-piperidin-N-oxyl, 2,2,6,6-Tetramethyl-piperidin-N-oxyl, U-vinul® 4040P der BASF SE, 4,4',4"-Tris-(2,2,6,6-tetramethyl-piperidin-N-oxyl)phosphit, 3-Oxo-2,2,5,5-tetramethyl-pyrrolidin-N-oxyl, 1-Oxyl-2,2,6,6-tetramethyl-4-methoxypiperidin, 1-Oxyl-2,2,6,6-tetramethyl-4-trimethylsilyloxypiperidin, 1-Oxyl-2,2,6,6-tetramethylpiperidin-4-yl-2-ethyl-hexanoat, 1-Oxyl-2,2,6,6-tetramethylpiperidin-4-yl-sebacat, 1-Oxyl-2,2,6,6-tetramethylpiperidin-4-yl-stearat, 1-Oxyl-2,2,6,6-tetramethylpiperidin-4-yl-benzoat, 1-Oxyl-2,2,6,6-tetramethylpiperidin-4-yl-(4-tert-butyl)benzoat, Bis-(1-oxyl-2,2,6,6-tetramethylpiperidin-4-yl)succinat, Bis-(1-oxyl-2,2,6,6-tetramethylpiperidin-4-yl)adipat, 1,10-Dekandisäure-bis-(1-oxyl-2,2,6,6-tetramethylpiperidin-4-yl)ester, Bis-(1-oxyl-2,2,6,6-tetramethylpiperidin-4-yl)n-butylmalonat, Bis-(1-oxyl-2,2,6,6-tetramethylpiperidin-4-yl)phthalat, Bis-(1-oxyl-2,2,6,6-tetramethylpiperidin-4-yl)isophthalat, Bis-(1-oxyl-2,2,6,6-tetramethylpiperidin-4-yl)terephthalat, Bis-(1-oxyl-2,2,6,6-tetramethylpiperidin-4-yl)hexahydroterephthalat, N,N'-Bis-(1-oxyl-2,2,6,6-tetramethylpiperidin-4-yl)adipinamid, N-(1-Oxyl-2,2,6,6-tetramethylpiperidin-4-yl)caprolactam, N-(1-Oxyl-2,2,6,6-tetramethylpiperidin-4-yl)dodecylsuccinimid, 2,4,6-Tris-[N-butyl-N-(1-oxyl-2,2,6,6-tetramethylpiperidin-4-yl]triazin, N,N'-Bis-(1-oxyl-2,2,6,6-tetramethylpiperidin-4-yl)-N,N'-bis-formyl-1,6-diaminohexan, 4,4'-Ethylen-bis-(1-oxyl-2,2,6,6-tetramethylpiperazin-3-on).

Bevorzugte N-Oxyl-gruppenhaltige Verbindungen sind 4-Hydroxy-2,2,6,6-tetramethyl-piperidin-N-oxyl, 4-Oxo-2,2,6,6-tetramethyl-piperidin-N-oxyl, 4-Methoxy-2,2,6,6-tetramethyl-piperidin-N-oxyl, 2,2,6,6-Tetramethyl-piperidin-N-oxyl, 4-Acetoxy-2,2,6,6-tetramethyl-piperidin-N-oxyl und 2,2,6,6-Tetramethyl-piperidin-N-oxyl, besonders bevorzugt ist 4-Hydroxy-2,2,6,6-tetramethyl-piperidin-N-oxyl.

Als Dicarbonsäure eignen sich alle unverzweigten und verzweigten Carbonsäuren mit zwei Carbonsäuregruppen, beispielsweise C₂-C₂₀-Alkandisäuren, bei denen im Falle der unverzweigten Dicarbonsäuren die Carbonsäuregruppen jeweils an den Enden der C₁-C₂₀-Alkankette sein können. Selbstverständlich können die Carbonsäuregruppen auch an einer beliebigen Stelle der Alkankette sein, so dass es sich dann um verzweigte Dicarbonsäuren handelt.

Bevorzugte Dicarbonsäuren sind die unverzweigten gesättigten Alkandisäuren mit einer C₂-C₁₆-Alkankette wie Ethandisäure (Oxalsäure), Propandisäure (Malonsäure), Butandisäure (Bernsteinsäure), Pentandisäure (Glutarsäure), Hexandisäure (Adipinsäure), Heptandisäure (Pimelinsäure), Octandisäure (Korksäure, Suberinsäure), Nonandisäure (Azelainsäure), Decandisäure (Sebacinsäure), Undecandisäure, Dodecandisäure, Tridecandisäure (Brassylsäure), Tetradecandisäure und Hexadecandisäure (Thapsiasäure). Besonders bevorzugt aus dieser Gruppe sind die Dicarbonsäuren mit einer C₆-C₁₄-Alkankette und ganz besonders bevorzugt mit einer C₈-C₁₂-Alkankette wie Octandisäure (Korksäure, Suberinsäure), Nonandisäure (Azelainsäure), Decandisäure (Sabacinsäure), Undecandisäure und Dodecandisäure. Insbesondere bevorzugt ist Sebacinsäure (Decandisäure).

Selbstverständlich können diese Alkandisäuren auch ein- oder mehrfach ungesättigt sein, beispielsweise Maleinsäure oder Fumarsäure. In Frage kommen ebenfalls ein- oder mehrfach substituierte Alkandisäuren wie beispielsweise Hydroxysubstituierte Alkandisäuren (Tartronsäure, Weinsäure, Äpfelsäure), Ketosubstituierte Alkandisäuren (α-Ketoglutarsäure, Oxalessigsäure) und Aminosubstituierte Alkandisäuren (Glutaminsäure, Asparaginsäure). Im Falle von ungesättigten Alkandisäuren können diese auch als aromatischer Ring vorliegen, wie beispielsweise Phthalsäure, Isophthalsäure und Terephthalsäure.

Die Dicarbonsäure und die N-Oxylgruppenhaltige Verbindung werden vom Fachmann so ausgewählt, dass der Diester der Dicarbonsäure mit den N-Oxyl-Gruppen einen Dampfdruck von weniger als 10⁻⁵ mbar, bevorzugt weniger als 10⁻⁶ mbar, besonders bevorzugt weniger als 10⁻⁸ mbar und ganz besonders bevorzugt weniger als 5*10⁻⁹ mbar aufweist.

Ein besonders bevorzugter Vertreter dieser Substanzklasse ist der Diester von Sebacinsäure mit 4-Hydroxy-2,2,6,6-tetramethylpiperidin-N-oxyl, der auch unter dem Handelsnamen Prostab^{®} 5415 von BASF SE erhältlich ist.

Beim Einsatz solcher Diester von Dicarbonsäuren, die mit N-Oxyl-gruppenhaltigen Verbindungen verestert sind, konnten im Rein-Propylheptyl(meth)acrylat weniger als 20 ppm, bevorzugt weniger als 10 ppm und besonders bevorzugt weniger als 5 ppm der N-Oxylverbindung nachgewiesen werden.

Alle der zuvor beschriebenen Polymerisationsinhibitoren, also Phenothiazin, 4-Nitrosophenol, 4-Hydroxy-2,2,6,6-tetramethylpiperidin-N-oxyl, Hydrochinon oder Hydrochinonmonomethylether sowie die Diester von Dicarbonsäuren, die mit N-Oxyl-gruppenhaltigen Verbindungen verestert sind, können allein oder in beliebiger Mischung sowohl in fester Form oder als Lösung dosiert werden. Bevorzugt werden sie als Lösung zugeführt.

Hierbei kommen als Lösungsmittel für den oder die Polymerisationsinhibitoren Rein-Propylheptyl(meth)acrylat, aber auch der entsprechende Rohester oder gewaschene Rohester (Neutralester) in Frage. Die Konzentration an Polymerisationsinhibitor in der Lösung beträgt zwischen 0,1 und 2,0 Gew.-%. Diese Lösung wird vorzugsweise direkt den jeweiligen Destillationskolonnen, beispielsweise über die Rücklaufleitung und/oder den Kondensatoren am Kolonnenkopf zugefahren.

Das Vakuum in den einzelnen Destillationskolonnen kann durch Dampfstrahler oder Flüssigkeitsringpumpen, die beispielsweise mit Wasser betrieben werden, erzeugt werden.

Die Rückstände aus der Schleppmitteldestillation sowie aus der Rückstandsdestillation können beispielsweise in einer geeigneten Verbrennungsanlage thermisch verwertet werden. Die aus der Anlage kommenden Abgase können beispielsweise in einer Fackel entsorgt werden.

Die Erfindung wird im Folgenden anhand eines Ausführungsbeispiels näher erläutert.

In die Verfahrensstufe A, die Veresterung, wird ein Strom 1, enthaltend Rein-(Meth)acrylsäure, ein Strom 2, enthaltend Propylheptanol-Isomere, ein Strom 3, enthaltend sauren Katalysator, ein Strom 4, enthaltend Polymerisationsinhibitor sowie ein Strom 5, enthaltend Schleppmittel, zugeführt.

Der Hauptstrom aus der Verfahrensstufe A wird in die Verfahrensstufe B, die Neutralisation geleitet, worin eine Neutralisation unter Zugabe einer Alkalilösung, Strom 6, erfolgt. Der Hauptstrom aus der Neutralisation wird in die Verfahrensstufe C, die Wäsche, geleitet, worin unter Zuführung einer Waschlösung, Strom 7, ein gewaschener Rohester erhalten wird, der anschließend in die Verfahrensstufe D, die Schleppmitteldestillation geleitet wird. Aus der Schleppmitteldestillation kann ein Schleppmittel enthaltender Strom in die Verfahrensstufe A, die Veresterung, recycliert werden. Der Hauptstrom aus der Schleppmitteldestillation wird in die Verfahrensstufe E, die Leichtsiederabtrennung, geleitet. Hieraus kann ein Teilstrom gegebenenfalls zusammen mit dem Teilstrom aus der Schleppmitteldestillation in die Verfahrensstufe A, die Veresterung, recycliert werden. Der Hauptstrom aus der Leichtsiederabtrennung, Verfahrensstufe E, wird in die Luftsättigung, Verfahrensstufe F, geleitet, in welcher ein sauerstoffhaltiges Gas, Strom 10, im Gegenstrom durchgeleitet wird. Der Sumpfstrom aus der Luftsättigung wird in die Reindestillation, Verfahrensstufe G, geleitet. Der Verfahrensstufe G wird ein Stabilisator, Strom 11, zugeführt und der Zielester, Rein-Propylheptyl(meth)acrylat, Strom 12, dampfförmig abgezogen. Der Sumpfstrom aus der Reindestillation wird in einer Rückstandsdestillation, Verfahrensstufe H, weiter aufgearbeitet, woraus ein Schwersiederrückstand, Strom 13, ausgeschleust wird.

Ein weiterer Gegenstand der vorliegenden Erfindung ist die Verwendung der genannten Diester von Dicarbonsäuren, bei denen beide Säuregruppen der Dicarbonsäure mit einer N-Oxyl-gruppenhaltigen Verbindung verestert sind, als Polymerisationsstabilisatoren in einer destillativen bzw. rektifikativen Aufreinigung von (Meth)acrylsäureestern mit einem Siedepunkt (bei Normaldruck) von > 150 °C, bevorzugt von > 180 °C, besonders bevorzugt von > 200 °C und ganz besonders bevorzugt von > 220 °C. Solche (Meth)acrylsäureester sind beispielsweise 2-Propylheptyl(meth)acrylat, Hydroxyalkyl(meth)acrylate wie 2-Hydroxyethyl(meth)acrylat und 4-Hydroxybutyl(meth)acrylat, Cyclohexyl(meth)acrylat und (Meth)acrylate von Fettalkoholen mit 12 bis 18 Kohlenstoffatomen sowie von Mischungen dieser Fettalkohole, wie beispielsweise eine Mischung aus Fettalkoholen mit einer C₁₆- und C₁₈-Alkylkette, mit einer C₁₃- und C₁₅-Alkylkette oder mit einer C₁₂- und C₁₄-Alkylkette. Beispiele solcher (Meth)acrylate von Fettalkoholen sind Lauryl(meth)acrylat, Cetyl(meth)acrylat und Stearyl(meth)acrylat.

Beim Einsatz solcher Diester von Dicarbonsäuren, die mit N-Oxyl-gruppenhaltigen Verbindungen verestert sind, können im Reinester weniger als 20 ppm, bevorzugt weniger als 10 ppm und besonders bevorzugt weniger als 5 ppm der N-Oxylverbindung nachgewiesen werden. Dadurch werden insbesondere qualitativ hochwertigen Zielester erhalten, da diese frei von verfärbenden Substanzen sind.

### Beispiele

### Beispiel 1

### Herstellung von 2-Propylheptylacrylat mit Luftsättigung (Verfahrensstufe F)

In einem Verfahren zur kontinuierlichen Herstellung von Propylheptylacrylat wurden dem ersten Reaktor einer dreistufigen Reaktorkaskade mit Naturumlaufverdampfern kontinuierlich 111 kg/h Acrylsäure mit einem Gehalt an Phenothiazin als Prozessstabilisator von 0,1 Gew.-%, bezogen auf das Gewicht der Acrylsäure, 2 kg/h einer 65 %igen wässrigen Lösung von Methansulfonsäure und 210 kg/h Propylheptanol-Isomere zudosiert. Die Edukte entsprachen jeweils der nachstehend angegebenen Qualität, wobei die Komponenten in Gew.-% angegeben sind: Acrylsäure mit 99,5 % Acrylsäure, 0,1 % Wasser, 0,13 % Diacrylsäure und 0,12 % Essigsäure, Propylheptanol-Isomere mit 93 % 2-Propylheptanol, 2,9 % 2-Propyl-4-methylhexanol, 3,9 % 2-Propyl-5-methylhexanol und 0,2 % Isopropylheptanol.

Darüber hinaus wurde dem ersten Reaktor der Kaskade kontinuierlich ein Kreislaufrückstrom vom Kopf der Schleppmitteldestillation (Verfahrensstufe D) von 130 kg/h und der nachfolgenden Zusammensetzung zugeführt: 22,57 Gew.-% Cyclohexan, 8,16 Gew.-% Propylheptanol-Isomere, 66,77 Gew.-% Propylheptylacrylat und1,36 Gew.-% Acrylsäure.

Der Destillationskolonne, die auf den ersten Reaktor der Kaskade aufgesetzt war, wurden 185 kg/h Cyclohexan als Schleppmittel zur Abtrennung des Veresterungswassers als Rücklauf aufgegeben.

Das molare Verhältnis Methacrylsäure/Propylheptanol-Isomere im Reaktorzulauf betrug 1,16 : 1.

In den Reaktoren der Kaskade stellte sich eine Temperatur von 120 °C ein.

Aus dem dritten Reaktor der Kaskade wurde ein Reaktionsaustrag von 598 kg/h mit der nachfolgend aufgeführten Zusammensetzung abzogen: 10 Gew.-% Cyclohexan, 2 Gew.-% Propylheptanol-Isomere, 83,8 Gew.-% Propylheptylacrylat, 2,1 Gew.-% Acrylsäure und 0,65 Gew.-% Methansulfonsäure.

Der Reaktoraustrag wurde auf eine Temperatur von 30 °C gekühlt und mit einer 20 %igen wässrigen Natriumcarbonatlösung in Verfahrensstufe B neutralisiert. Das molare Verhältnis Natriumcarbonat zu Acrylsäure + Methansulfonsäure betrug 3,5 : 1.

Der in Verfahrensstufe B erhaltene Neutralester wurde in Verfahrensstufe C mit 1200 kg/h Wasser in einer Mixer-Settler-Apparatur von restlichen Salzen befreit und gewaschen. Der gewaschene Neutralester wurde der Verfahrensstufe D, der Schleppmitteldestillation, zugeführt. Das an Cyclohexan angereicherte Destillat wurde in die Blase des ersten Veresterungsreaktors zugeführt.

Die Schleppmitteldestillation wurde bei einem Kopfdruck von 60 mbar und einer Sumpftemperatur von 85 °C in einer mit Pallringen der Größe 35 bestückten Rektifikationskolonne durchgeführt.

Der Sumpfaustrag aus der Schleppmitteldestillation wurde in einer mit Pallringen der Größe 25 bestückten Destillationskolonne in Verfahrensstufe E, der Leichtsiederabtrennung, bei einem Kopfdruck von 15 mbar und einer Sumpftemperatur von 140 °C destilliert.

Das Destillat, das neben den Propylheptanol-Isomeren hauptsächlich Propylheptylacrylat enthielt, wurde in den ersten Reaktor der Veresterungskaskade zurückgeführt. Optional hätte ein Teil der Leichtsieder ausgeschleust werden können.

In Verfahrensstufe F, der Luftsättigung wurde der aus der Leichtsiederabtrennung (Verfahrensstufe E) erhaltene Rohester im Gegenstrom mit Magerluft beaufschlagt. Hierzu wurden 330 kg/h Rohester auf den Kopf einer mit Pallringen der Größe 25 bestückten Kolonne aufgegeben. Im unteren Teil der Kolonne wurde 1 m³/h Magerluft mit einem sauerstoffgehalt von 6 Vol.-% aufgegeben. Die Temperatur in der Kolonne betrug 25 °C.

In der Verfahrensstufe G, der Reindestillation, wurden in einem ersten Dünnschichtverdampfer bei einem Eindampfverhältnis (Verhältnis von Brüden- zu Feedstrom) von ca. 75 % ein Kopfstrom von 275 kg/h Rein-Propylheptylacrylat mit der folgenden Zusammensetzung gewonnen:
99,39 Gew.-% Propylheptylacrylat 0,16 Gew.-% Propylheptylacetat, 0,11 Gew.-% Propylheptyldiacrylsäureester und 0,22 Gew.-% Propylheptanol. Das Rein-Propylheptylacrylat wurde durch Zugabe von Hydrochinonmonomethylether (MEHQ) in einer Konzentration von 250 ppm für die Lagerung gegen Polymerisation stabilisiert.

### Beispiel 2

Beispiele zur Stabilisierung der Reindestillation (Verfahrensstufe G) von Propylheptylacrylat Die folgenden Beispielen 2a bis 2c wurde analog zu Beispiel 1 durchgeführt.

### Beispiel 2a (Vergleichsbeispiel)

Reindestillation von Propylheptylacrylat mit PTZ/MEHQ ohne Luftsättigung (Verfahrensstufe F)

367 kg/h Roh-Propylheptylacrylat aus der Leichtsiederabtrennung (Verfahrensstufe E) wurden mit Phenothiazin (PTZ) und Hydrochinonmonomethylether (MEHQ) auf einen Gehalt von 200 ppm PTZ und 200 ppm MEHQ aufstabilisiert. Die Destillation im Dünnschichtverdampfer wurde bei einer Kopftemperatur von 96 °C und einem Kopfdruck von 5 mbar durchgeführt. Es wurden dabei 275 kg/h Rein-Propylheptylacrylat erhalten. Nach ca. 20 stunden setzte Polymerisation ein, die Destillationseinheit musste abgestellt und gereinigt werden.

### Beispiel 2b

Reindestillation von Propylheptylacrylat mit PTZ/MEHQ mit Luftsättigung (Verfahrensstufe F) 367 kg/h Rohpropylheptylacrylat aus der Luftsättigung (Verfahrensstufe F) wurden mit PTZ und MEHQ auf einen Gehalt von 200 ppm PTZ und 200 ppm MEHQ aufstabilisiert. Die Destillation im Dünnschichtverdampfer wurde bei einer Kopftemperatur von 99 °C und einem Kopfdruck von 6 mbar durchgeführt. Es wurden dabei 275 kg/h Rein-Propylheptylacrylat erhalten. Die Destillation lief 48 Stunden ohne Polymerisation in der Destillationseinheit. Ein Teil des Stabilisators MEHQ geht bedingt durch dessen Flüchtigkeit (Dampfdruck ca. 1,4 Pa bei 25 °C, ca. 370 Pa bei 100 °C) bei der Destillation über Kopf in das Reinprodukt. Die Dosierung des Stabilisators MEHQ in das Roh-Propylheptylacrylat ist somit schwierig einzustellen, da Teile davon mitdestilliert werden.

### Beispiel 2c

### Reindestillation von Propylheptylacrylat mit PTZ/Prostab^{®} 5415 mit Luftsättigung

356 kg/h Roh-Propylheptylacrylat aus der Luftsättigung (Verfahrensstufe F) wurden mit PTZ und einem Diester von Sebacinsäure und 4-Hydroxy-2,2,6,6-tetramethylpiperidin-N-oxyl (erhältlich unter dem Handelsnamen Prostab^{®} 5415 von der BASF SE) auf einen Gehalt von 200 ppm PTZ und 200 ppm Prostab^{®} 5415 aufstabilisiert. Die Destillation im Dünnschichtverdampfer wurde bei einer Kopftemperatur von 99 °C und einem Kopfdruck von 6 mbar durchgeführt. Es wurden 275 kg/h Rein-Propylheptylacrylat erhalten. Die Destillation lief > 96 Stunden ohne Polymerisation in der Destillationseinheit. Im Rein-Propylheptylacrylat konnten keine N-Oxylverbindungen nachgewiesen werden.

### Beispiel 3

### Beispiele zur Stabilisierung der Reindestillation (Verfahrensstufe G) von 4-Hydroxybutylacrylat (4-HBA)

Propylheptylacrylat und 4-HBA weisen ähnliche Siedepunkte auf (Propylheptylacrylat: 250 °C bei 10⁵ Pa, 4-HBA: 230 °C bei 10⁵ Pa). Zur Verbesserung der Stabilität wurde während der Reindestillation (Verfahrensstufe G) anstelle von MEHQ 4-Hydroxy-2,2,6,6-tetramethylpiperidin-N-oxyl (HOT) eingesetzt.

Die folgenden Beispielen 3a und 3b wurde ansonsten analog zu Beispiel 1 durchgeführt.

### Beispiel 3a (Vergleichsbeispiel)

### Reindestillation von 4-HBA mit PTZ/HOT mit Luftsättigung (Verfahrensstufe F)

367 kg/h Roh-4-Hydroxybutylacrylat aus der Luftsättigung (Verfahrensstufe F) wurden mit PTZ und HOT auf einen Gehalt von 200 ppm PTZ und 200 ppm HOT aufstabilisiert. Die Destillation im Dünnschichtverdampfer wurde bei einer Kopftemperatur von 102 °C und einem Kopfdruck von 5 mbar durchgeführt. Es wurden dabei 280 kg/h Rein-4-Hydroxybutylacrylat erhalten. Die Destillation lief > 96 Stunden ohne Polymerisation in der Destillationseinheit. Im Rein-4-Hydroxybutylacrylat wurden bis zu 65 ppm der N-Oxylverbindung nachgewiesen. Diese sind im 4-HBA jedoch unerwünscht, da sie zu Verfärbungen bei Polymeren enthaltend 4-HBA führen können. Derartige Verfärbungen sind insbesondere im Lackbereich unerwünscht.

### Beispiel 3b

### Reindestillation von 4-HBA mit PTZ/Prostab^{®} 5415 mit Luftsättigung (Verfahrensstufe F)

356 kg/h Roh-4-Hydroxybutylacrylat aus der Luftsättigung (Verfahrensstufe F) wurden mit PTZ und einem Diester von Sebacinsäure und 4-Hydroxy-2,2,6,6-tetramethylpiperidin-N-oxyl (erhältlich unter dem Handelsnamen Prostab^{®} 5415 von der BASF SE) auf einen Gehalt von 200 ppm PTZ und 200 ppm Prostab^{®} 5415 aufstabilisiert. Die Destillation im Dünnschichtverdampfer wurde bei einer Kopftemperatur von 102 °C und einem Kopfdruck von 5 mbar durchgeführt. Es wurden 270 kg/h Rein-4-Hydroxybutylacrylat erhalten. Die Destillation lief > 96 Stunden ohne Polymerisation in der Destillationseinheit. Im Rein-4-Hydroxybutylacrylat konnten keine N-Oxylverbindungen nachgewiesen werden.

## Patentansprüche

1. Kontinuierliches Verfahren zur Herstellung von (Meth)acrylaten von C₁₀-Alkoholgemischen durch Umsetzung von Rein-(Meth)acrylsäure mit einem Isomerengemisch von C₁₀-Alkoholen aus 2-Propylheptanol als Hauptisomer und mindestens einem der C₁₀-Alkohole 2-Propyl-4-methylhexanol, 2-Propyl-5-methylhexanol, 2-Isopropylheptanol, 2-Isopropyl-4-methylhexanol, 2-isopropyl-5-methylhexanol und/oder 2-Propyl-4,4-dimethylpentanol, in Gegenwart mindestens eines sauren Katalysators und mindestens eines Polymerisationsinhibitors und in Gegenwart eines Schleppmittels für das Veresterungswasser, mit folgenden Verfahrensschritten:
- Veresterung der Rein-(Meth)acrylsäure (1) mit dem Isomerengemisch aus Propylheptanol-Isomeren (2) in Gegenwart des sauren Katalysators (3), des Polymerisationsinhibitors (4) sowie des Schleppmittels für das Veresterungswasser (5) in einer Reaktionszone (A), wobei das Veresterungswasser in einer der Reaktionszone (A) aufgesetzten Destillationszone als Azeotrop mit dem Schleppmittel abgezogen wird und wobei ein Reaktionsaustrag erhalten wird (Verfahrensstufe A), der
- einer Neutralisation zugeführt wird, wobei aus dem Reaktionsaustrag aus der Veresterung mittels einer alkalischen Lösung der saure Katalysator (3) sowie nicht umgesetzte Rein-(Meth)acrylsäure (1) neutralisiert werden unter Erhalt von Roh-Propylheptyl(meth)acrylat (Verfahrensstufe B),
- Wäsche des Roh-Propylheptyl(meth)acrylats aus Verfahrensstufe B, wobei Reste von Salzen aus dem Roh-Propylheptyl(meth)acrylat abgetrennt werden (Verfahrensstufe C),
- Schleppmitteldestillation des gewaschenen Roh-Propylheptyl(meth)acrylats aus Verfahrensstufe C unter vermindertem Druck und unter kontinuierlicher Zudosierung mindestens eines Polymerisationsinhibitors, wobei das Schleppmittel, andere Leichtsieder sowie ein sehr kleiner Anteil an Propylheptyl(meth)acrylat abgezogen werden (Verfahrensstufe D),
- Leichtsiederabtrennung aus dem Sumpfstrom aus der Verfahrensstufe D unter vermindertem Druck und kontinuierlicher Zudosierung mindestens eines Polymerisationsinhibitors zur Abtrennung der Reste an Leichtsiedern sowie eines kleinen Anteils des Propylheptyl(meth)acrylats (Verfahrensstufe E),
- Luftsättigung des Sumpfstroms aus Verfahrensstufe E im Gegenstrom durch Einblasen eines sauerstoffhaltigen Gasgemisches und Abtrennung des entstehenden Abgases(Verfahrensstufe F),
- Reindestillation des Sumpfstroms aus der Verfahrensstufe F unter vermindertem Druck und kontinuierlicher Zudosierung mindestens eines Polymerisationsinhibitors unter Erhalt von Rein-Propylheptyl(meth)acrylat sowie eines Sumpfstromes, enthaltend die Polymerisationsinhibitoren sowie Hochsieder (Verfahrensstufe G);
- Rückstandsdestillation des Sumpfstroms aus der Verfahrensstufe G unter vermindertem Druck und kontinuierlicher Zudosierung mindestens eines Polymerisationinhibitors, wobei restliche Anteile an Propylheptyl(meth)acrylat von Polymerisationsinhibitoren sowie Hochsiedern getrennt werden (Verfahrensstufe H).

2. Verfahren nach Anspruch 1, **dadurch gekennzeichnet, dass** das sauerstoffhaltige Gas in der Luftsättigung (Verfahrensstufe F) Sauerstoff, Luft oder Magerluft ist.

3. Verfahren nach Anspruch 2, **dadurch gekennzeichnet, dass** der Sauerstoffgehalt des sauerstoffhaltigen Gases zwischen 1 und 21 Vol.-%, bezogen auf das Gesamtvolumen des sauerstoffhaltigen Gases, beträgt.

4. Verfahren nach einem der Ansprüche 2 oder 3, **dadurch gekennzeichnet, dass** der Sauerstoffgehalt des sauerstoffhaltigen Gases zwischen 4 und 8 Vol.-%, bezogen auf das Gesamtvolumen des sauerstoffhaltigen Gases, beträgt.

5. Verfahren nach einem der vorstehenden Ansprüche, **dadurch gekennzeichnet, dass** in der Luftsättigung (Verfahrensstufe F) die Luft im Gegenstrom zur das Rohprodukt enthaltenden Flüssigkeit mit einer Geschwindigkeit von 0,1 bis 10 m³/h eingeleitet wird, wobei sich diese Geschwindigkeitswerte auf eine Magerluft mit ca. 6 Vol.-% Sauerstoffgehalt beziehen.

6. Verfahren nach Anspruch 5, **dadurch gekennzeichnet, dass** die Luft im Gegenstrom zur das Rohprodukt enthaltenden Flüssigkeit mit einer Geschwindigkeit von 0,5 bis 5 m³/h eingeleitet wird, wobei sich diese Geschwindigkeitswerte auf eine Magerluft mit ca. 6 Vol.-% Sauerstoffgehalt beziehen.

7. Verfahren nach einem der vorstehenden Ansprüche, **dadurch gekennzeichnet, dass** in der Luftsättigung (Verfahrensstufe F) die das Rohprodukt enthaltende Flüssigkeit mit einer Geschwindigkeit von 300 bis 600 kg/h eingeleitet wird.

8. Verfahren nach einem der vorstehenden Ansprüche, **dadurch gekennzeichnet, dass** in der Luftsättigung (Verfahrensstufe F) als Apparate Füllkörperkolonnen, Packungskolonnen oder Bodenkolonnen eingesetzt werden.

9. Verfahren nach einem der vorstehenden Ansprüche, **dadurch gekennzeichnet, dass** als Polymerisationsinhibitoren Phenothiazin, 4-Nitrosophenol, 4-Hydroxy-2,2,6,6-tetramethylpiperidin-N-oxyl, Hydrochinon oder Hydrochinonmonomethylether, sowie Diester von Dicarbonsäuren, die mit N-Oxyl-gruppenhaltigen Verbindungen verestert sind, allein oder in beliebiger Mischung eingesetzt werden.

10. Verfahren nach Anspruch 9, **dadurch gekennzeichnet dass**, die Diester von Dicarbonsäuren , die mit N-Oxyl-gruppenhaltigen Verbindungen verestert sind, einen Dampfdruck von weniger als 10⁻⁵ mbar, aufweisen.

11. Verfahren nach Anspruch 9, **dadurch gekennzeichnet, dass** die Diester einen Dampfruck von weniger als 10⁻⁶ mbar aufweisen.

12. Verfahren nach einem der Ansprüche 9 bis 11, **dadurch gekennzeichnet, dass** die N-Oxylgruppenhaltige Verbindung 4-Hydroxy-2,2,6,6-tetramethylpiperidin-N-oxyl ist.

13. Verfahren nach einem der Ansprüche 9 bis 12, **dadurch gekennzeichnet dass** der Diester von Sebacinsäure mit 4-Hydroxy-2,2,6,6-tetramethylpiperidin-N-oxyl ist.

14. Verwendung eines Diesters gemäß Ansprüchen 9 bis 13 als Polymerisationsinhibitoren in einer destillativen bzw. rektifikativen Aufreinigung von (Meth)acrylsäureestern mit einem Siedepunkt (bei Normaldruck) von > 150 °C.

15. Verwendung nach Anspruch 14, **dadurch gekennzeichnet, dass** der (Meth)acrylsäureester ausgewählt ist aus der Gruppe von Propylheptyl(meth)acrylat, Hydroxyalkyl(meth)acrylate wie 2-Hydroxyethyl(meth)acrylat und 4-Hydroxybutyl(meth)acrylat, Cyclohexyl(meth)acrylat und (Meth)acrylate von Fettalkoholen mit 12 bis 18 Kohlenstoffatomen sowie von Mischungen dieser Fettalkohole.

## Claims

1. A continuous process for preparing (meth)acrylates of C₁₀-alcohol mixtures by reacting glacial (meth) acrylic acid with an isomer mixture of C₁₀-alcohols composed of 2-propylheptanol as the main isomer and at least one of the C₁₀-alcohols 2-propyl-4-methylhexanol, 2-propyl-5-methyl-hexanol, 2-isopropylheptanol, 2-isopropyl-4-methylhexanol, 2-isopropyl-5-methylhexanol and/or 2-propyl-4,4-dimethylpentanol, in the presence of at least one acidic catalyst and at least one polymerization inhibitor and in the presence of an azeotroping agent for the water of esterification, comprising the following process steps:
- esterifying the glacial (meth)acrylic acid (1) with the isomer mixture of propylheptanol isomers (2) in the present of the acidic catalyst (3), the polymerization inhibitor (4) and the azeotroping agent for the water of esterification (5) in a reaction zone (A), the water of esterification being drawn off in a distillation zone connected atop the reaction zone (A) as an azeotrope with the azeotroping agent, giving a reaction output (process stage A), which
- is supplied to a neutralization, with neutralization of the acidic catalyst (3) and unconverted glacial (meth)acrylic acid (1) from the reaction output from the esterification by means of an alkaline solution to obtain crude propylheptyl (meth)acrylate (process stage B),
- washing the crude propylheptyl (meth)acrylate from process stage B to remove residues of salts from the crude propylheptyl (meth)acrylate (process stage C),
- azeotroping agents distillation of the washed crude propylheptyl (meth)acrylate from process stage C under reduced pressure and with continuous metered addition of at least one polymerization inhibitor, and drawing off the azeotroping agent, other low boilers and a very small proportion of propylheptyl (meth)acrylate (process stage D),
- low boiler removal from the bottom stream from process stage D under reduced pressure and with continuous metered addition of at least one polymerization inhibitor for removal of the residues of low boilers and of a small proportion of the propylheptyl (meth)acrylate (process stage E),
- air saturation of the bottom stream from process stage E in countercurrent by blowing in an oxygenous gas mixture and removing the resulting offgas (process stage F),
- purifying distillation of the bottom stream from process stage F under reduced pressure and with continuous metered addition of at least one polymerization inhibitor to obtain glacial propylheptyl (meth)acrylate and a bottom stream comprising the polymerization inhibitors and high boilers (process stage G);
- residue distillation of the bottom stream from process stage G under reduced pressure and with continuous metered addition of at least one polymerization inhibitor, with separation of residual proportions of propylheptyl (meth)acrylate from polymerization inhibitors and high boilers (process stage H).

2. The process according to claim 1, wherein the oxygenous gas in the air saturation (process stage F) is oxygen, air or lean air.

3. The process according to claim 2, wherein the oxygen content of the oxygenous gas is between 1 and 21% by volume, based on the total volume of the oxygenous gas.

4. The process according to either of claims 2 and 3, wherein the oxygen content of the oxygenous gas is between 4 and 8% by volume, based on the total volume of the oxygenous gas.

5. The process according to any of the preceding claims, wherein the air in the air saturation (process stage F) is introduced in countercurrent to the liquid comprising the crude product at a rate of 0.1 to 10 m³/h, these rate values being based on lean air having oxygen content approx. 6% by volume.

6. The process according to claim 5, wherein the air is introduced in countercurrent to the liquid comprising the crude product at a rate of 0.5 to 5 m³/h, these rate values being based on lean air having oxygen content approx. 6% by volume.

7. The process according to any of the preceding claims, wherein the liquid comprising the crude product is introduced in the air saturation (process stage F) at a rate of 300 to 600 kg/h.

8. The process according to any of the preceding claims, wherein the apparatuses used in the air saturation (process stage F) are columns with random packing, columns with structured packing or tray columns.

9. The process according to any of the preceding claims, wherein the polymerization inhibitors used are phenothiazine, 4-nitrosophenol, 4-hydroxy-2,2,6,6-tetramethylpiperidine N-oxyl, hydroquinone or hydroquinone monomethyl ether, and diesters of dicarboxylic acids which have been esterified with N-oxyl-containing compounds, alone or in any desired mixture.

10. The process according to claim 9, wherein the diesters of dicarboxylic acids which have been esterified with N-oxyl-containing compounds have a vapor pressure of less than 10⁻⁵ mbar.

11. The process according to claim 9, wherein the diesters have a vapor pressure of less than 10⁻⁶ mbar.

12. The process according to any of claims 9 to 11, wherein the N-oxyl-containing compound is 4-hydroxy-2,2,6,6-tetramethylpiperdine N-oxyl.

13. The process according to any of claims 9 to 12, wherein the diester is of sebacic acid with 4-hydroxy-2,2,6,6-tetramethylpiperidine N-oxyl.

14. The use of a diester according to claims 9 to 13 as polymerization inhibitors in a distillative or rectificative purification of (meth)acrylic esters having a boiling point (at standard pressure) of > 150°C.

15. The use according to claim 14, wherein the (meth)acrylic ester is selected from the group of propylheptyl (meth)acrylate, hydroxyalkyl (meth)acrylates such as 2-hydroxyethyl (meth)acrylate and 4-hydroxybutyl (meth)acrylate, cyclohexyl (meth)acrylate and (meth)acrylates of fatty alcohols having 12 to 18 carbon atoms, and of mixtures of these fatty alcohols.

## Revendications

1. Procédé continu pour la préparation de (méth)acrylates de mélanges d'alcools en C₁₀ par transformation d'acide (méth)acrylique pur avec un mélange d'isomères d'alcools en C₁₀ constitué par du 2-propylheptanol comme isomère principal et au moins un des alcools en C₁₀ parmi le 2-propyl-4-méthylhexanol, le 2-propyl-5-méthylhexanol, le 2-isopropylheptanol, le 2-isopropyl-4-méthylhexanol, le 2-isopropyl-5-méthylhexanol et/ou le 2-propyl-4,4-diméthylpentanol, en présence d'au moins un catalyseur acide et d'au moins un inhibiteur de polymérisation et en présence d'un agent d'entraînement pour l'eau d'estérification, présentant les étapes de procédé suivantes :
- estérification de l'acide (méth)acrylique pur (1) avec le mélange d'isomères de propylheptanol (2) en présence du catalyseur acide (3), de l'inhibiteur de polymérisation (4) ainsi que de l'agent d'entraînement pour l'eau d'estérification (5) dans une zone de réaction (A), l'eau de réaction étant soutirée dans une zone de distillation placée sur la zone de réaction (A) sous forme d'azéotrope avec l'agent d'entraînement et un produit de réaction étant obtenu (étape de procédé A), qui
- est introduit dans une neutralisation, le catalyseur acide (3) ainsi que l'acide (méth)acrylique pur (1) non transformé du produit de réaction provenant de l'estérification étant neutralisés par une solution alcaline avec obtention de (méth)acrylate de propylheptyle brut (étape de procédé B) ;
- lavage du (méth)acrylate de propylheptyle brut de l'étape de procédé B, les restes de sels étant séparés du (méth)acrylate de propylheptyle brut (étape de procédé C) ;
- distillation avec un agent d'entraînement du (méth)acrylate de propylheptyle brut lavé provenant de l'étape de procédé C sous pression réduite et avec dosage continu d'au moins un inhibiteur de polymérisation, l'agent d'entraînement, d'autres fractions à bas point d'ébullition ainsi qu'une très petite proportion de (méth)acrylate de propylheptyle étant soutirés (étape de procédé D) ;
- séparation des fractions à bas point d'ébullition du flux de fond provenant de l'étape de procédé D sous pression réduite et avec dosage continu d'au moins un inhibiteur de polymérisation pour la séparation des restes de fractions à bas point d'ébullition ainsi que d'une petite proportion de (méth)acrylate de propylheptyle (étape de procédé E) ;
- saturation à l'air du flux du fond de l'étape de procédé E à contre-courant par insufflation d'un mélange gazeux contenant de l'oxygène et séparation de l'effluent gazeux qui se forme (étape de procédé F) ;
- distillation pure du flux du fond de l'étape de procédé F sous pression réduite et avec dosage continu d'au moins un inhibiteur de polymérisation avec obtention de (méth)acrylate de propylheptyle pur et d'un flux de fond contenant les inhibiteurs de polymérisation ainsi que les fractions à point d'ébullition élevé (étape de procédé G) ;
- distillation du résidu du flux de fond de l'étape de procédé G sous pression réduite et avec dosage continu d'au moins un inhibiteur de polymérisation, les proportions résiduelles de (méth)acrylate de propylheptyle étant séparées des inhibiteurs de polymérisation et des fractions à point d'ébullition élevé (étape de procédé H).

2. Procédé selon la revendication 1, **caractérisé en ce que** le gaz contenant de l'oxygène dans la saturation à l'air (étape de procédé F) est de l'oxygène, de l'air ou de l'air maigre.

3. Procédé selon la revendication 2, **caractérisé en ce que** la teneur en oxygène du gaz contenant de l'oxygène se situe entre 1 et 21% en volume par rapport au volume total du gaz contenant de l'oxygène.

4. Procédé selon l'une quelconque des revendications 2 ou 3, **caractérisé en ce que** la teneur en oxygène du gaz contenant de l'oxygène se situe entre 4 et 8% en volume par rapport au volume total du gaz contenant de l'oxygène.

5. Procédé selon l'une quelconque des revendications précédentes, **caractérisé en ce que** dans la saturation à l'air (étape de procédé F), l'air est introduit à contre-courant par rapport au liquide contenant le produit brut à une vitesse de 0,1 à 10 m³/h, les valeurs de vitesse se rapportant à un air maigre présentant une teneur en oxygène d'environ 6% en volume.

6. Procédé selon la revendication 5, **caractérisé en ce que** l'air est introduit à contre-courant par rapport au liquide contenant le produit brut à une vitesse de 0,5 à 5 m³/h, les valeurs de vitesse se rapportant à un air maigre présentant une teneur en oxygène d'environ 6% en volume.

7. Procédé selon l'une quelconque des revendications précédentes, **caractérisé en ce que** dans la saturation à l'air (étape de procédé F), le liquide contenant le produit brut est introduit à une vitesse de 300 à 600 kg/h.

8. Procédé selon l'une quelconque des revendications précédentes, **caractérisé en ce qu'**on utilise, dans la saturation à l'air (étape de procédé F), comme appareil, des colonnes à corps de remplissage, des colonnes garnies ou des colonnes à plateaux.

9. Procédé selon l'une quelconque des revendications précédentes, **caractérisé en ce qu'**on utilise, comme inhibiteurs de polymérisation, de la phénothiazine, du 4-nitrosophénol, du 4-hydroxy-2,2,6,6-tétraméthylpipéridin-N-oxyle, de l'hydroquinone ou de l'hydroquinonemonométhyléther, ainsi que des diesters d'acides dicarboxyliques, qui sont estérifiés par des composés contenant des groupes N-oxyle, seuls ou dans un mélange quelconque.

10. Procédé selon la revendication 9, **caractérisé en ce que** les diesters d'acide dicarboxylique, qui sont estérifiés par des composés contenant des groupes N-oxyle, présentent une tension de vapeur inférieure à 10⁻⁵ mbar.

11. Procédé selon la revendication 9, **caractérisé en ce que** les diesters présentent une tension de vapeur inférieure à 10⁻⁶ mbar.

12. Procédé selon l'une quelconque des revendications 9 à 11, **caractérisé en ce que** le composé contenant des groupes N-oxyle est le 4-hydroxy-2,2,6,6-tétraméthylpipéridin-N-oxyle.

13. Procédé selon l'une quelconque des revendications 9 à 12, **caractérisé en ce que** le diester est celui de l'acide sébacique avec du 4-hydroxy-2,2,6,6-tétraméthylpipéridin-N-oxyle.

14. Utilisation d'un diester selon les revendications 9 à 13 comme inhibiteur de polymérisation dans une purification par distillation ou rectification d'esters de l'acide (méth)acrylique présentant un point d'ébullition (à pression normale) > 150°C.

15. Utilisation selon la revendication 14, **caractérisée en ce que** l'ester de l'acide (méth)acrylique est choisi dans le groupe formé par le (méth)acrylate de propylheptyle, les (méth)acrylates d'hydroxyalkyle, tels que le (méth)acrylate de 2-hydroxyéthyle et le (méth)acrylate de 4-hydroxybutyle, le (méth)acrylate de cyclohexyle et les (méth)acrylates d'alcools gras comprenant 12 à 18 atomes de carbone ainsi que de mélanges de ces alcools gras.
